# EUROPÄISCHE PATENTANMELDUNG

(11) **EP 0 671 169 A1**
(43) Veröffentlichungstag der Anmeldung: **13.09.1995**
(21) Anmeldenummer: 95103045.1
(22) Anmeldetag: 03.03.1995
(51) Int. Cl.: A61K 9/51, A61K 47/48

(54) **Nanopartikel, enthaltend einen Wirkstoff und ein Polyketalweinsäureamid, Verfahren zu ihrer Herstellung und Verwendung derselben**

(30) Priorität: 09.03.1994 DE 4407898
(71) Anmelder: HOECHST AKTIENGESELLSCHAFT, D-65929 Frankfurt am Main (DE)
(72) Erfinder: Ahlers, Michael, Dr., D-55127 Mainz (DE); Walch, Axel, Dr., D-60487 Frankfurt (DE); Seipke, Gerhard, Dr., D-65719 Hofheim (DE); Russell-Jones, Gregory, Dr., Middel Cove, NSW 2068 (AU)

(57) **Zusammenfassung**

Nanopartikel, enthaltend einen Wirkstoff und ein Polyketalsäureamid eignen sich als Arzneimittelträger für Wirkstoffe, insbesondere für Peptide und Proteine. Es werden Verfahren zur Herstellung der Nanopartikel beschrieben.

## Beschreibung

Die Erfindung betrifft Nanopartikel, enthaltend einen Wirkstoff und ein Polyketalweinsäureamid, die sich als Arzneimittelträger für Wirkstoffe, insbesondere für Peptide oder Proteine, eignen.

In biologisch abbaubaren Arzneimittelabgabesystemen, wie in US 4,093,709 angegeben, wird der Wirkstoff in einem bioabbaubaren Polymeren dispergiert, welches beim Abbau den Wirkstoff freigibt. Typische, nach dem Stand der Technik meist untersuchte, biologisch abbaubare Polymere sind Homo- und Copolyester, insbesondere der Milch- und Glykolsäure, wie sie in US 3,773,919 und US 3,297,033 beschrieben sind. Nachteilig sind u. a. die schlecht kontrollierbare Quellbarkeit der Polyester im physiologischen Milieu und der damit verbundene komplexe Mechanismus der Wirkstofffreisetzung. Zusätzlich wird im allgemeinen nach einem erheblichen "initial burst" nur eine geringe bis mäßige Freisetzungsrate bewirkt.

In EP 0 514 790 werden bereits Polyketalweinsäureamide beschrieben. Mit den dort beschriebenen Verfahren gelingt es jedoch nicht Nanopartikel herzustellen, die einen Wirkstoff enthalten.

Orale Applikationsformen sollten neben dem Schutz vor Hydrolyse und einem geeigneten Freisetzungsprofil auch die Resorption des Wirkstoffs durch die Darmwand fördern. Eine zielgerichtete Freisetzung im Darmlumen aus Kapseln, die magensaftresistent sind, verhindert zwar die saure Hydrolyse im Magen, kann aber dazu führen, daß Peptid- und Protein-Wirkstoffe dem Abbau durch Verdauung unterliegen. Beispiele für solche Freisetzungsformen sind Kapseln aus Polyacrylderivaten mit pH-abhängigen Überzügen (Rubinstein et al. Int. J. Pharm. 30, 95-99, 1986) sowie Kapseln mit azoaromatischen Filmüberzügen, die bakteriell abgebaut werden (Saffran et al. Science, 233, 1081-1084, 1986).

Eine moderne Arzneimitteltherapie erfordert eine Darreichungsform, die eine kontrollierte Wirkstofffreigaberate gewährleistet. Besonders für hoch wirksame Peptid- und Proteinwirkstoffe ist eine gleichbleibende Freigabe über lange Zeiträume erforderlich.

Aufgabe der vorliegenden Erfindung ist es Nanopartikel herzustellen, die bioverträglich und abbaubar sind und sich als Träger, insbesondere für Peptid- und Protein-Wirkstoffe, eignen und als Freigabe- und Transportsystem dienen.

Es wurde nun gefunden, daß sich aus Polyketalweinsäureamiden der Formel I und Wirkstoffen Nanopartikel herstellen lassen, die geeignet sind für die Verwendung als Transport- und Freigabesysteme zur Applikation von Arzneimitteln.

Überraschenderweise gelang die Formulierung von wirkstoffenthaltenden Nanopartikeln aus diesen Polymeren als eine Voraussetzung für die gastrointestinale, unspezifische Resorption. Zusätzlich ist es möglich, diese wirkstoffenthaltenden Nanopartikel mit Kopplungsgruppen zu funktionalisieren, an die spezifischen Liganden gebunden werden können, die einen rezeptorvermittelten Transport durch die Darmwand ermöglichen. Spezifische Liganden lassen sich auch direkt in die Nanopartikeloberfläche einbauen.

Die Erfindung betrifft daher Nanopartikel, enthaltend einen Wirkstoff und ein Polyketalweinsäureamid, das im wesentlichen mindestens 95 Mol-% an wiederkehrenden Struktureinheiten der Formel I enthält, 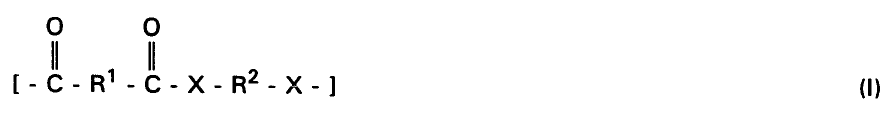
wobei R¹ für den Rest der Formel II 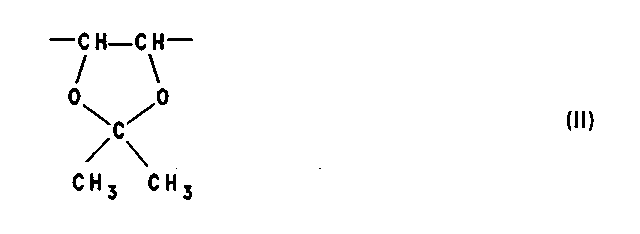
steht,
X für -NH- steht, und
R² für geradkettiges oder verzweigtes Alkyl oder Cycloalkyl steht, das mit einem oder mehreren inerten Resten substituiert sein kann.

Unter dem Begriff "inerter Rest" werden Substituenten verstanden, die unter den Herstellungs- und Verarbeitungsbedingungen der Polyketalweinsäureamide nicht miteinander reagieren oder durch Schutzgruppen daran gehindert werden miteinander zu reagieren. Inerte Reste können beispielsweise anorganische Reste, wie Halogen sein, oder es kann sich um organische Reste wie Alkyl, Alkenyl, Cycloalkyl, Cycloalkenyl, Alkoxy, oder Dialkylaminoalkyl handeln.

Funktionelle Gruppen, die durch Schutzgruppen an der Reaktion gehindert werden, sind beispielsweise Amino oder Hydroxy.

Bekannte Schutzgruppen sind die Benzyloxy- oder Phenylsulfonylgruppe. Unter dem Begriff Wirkstoff werden Wirkstoffe auf der Basis von Kohlehydraten, Peptiden und Proteinen verstanden, z.B. Insulin, Calcitonin oder Buserelin.

Nanopartikel sind kugelförmige Körper mit einem Durchmesser von 10 bis 1000 nm, bevorzugt von 50 bis 800 nm, insbesondere von 200 bis 600 nm.

Bevorzugt werden in den Nanopartikeln Polyketalweinsäureamide der Formel I eingesetzt, die im wesentlichen mindestens 95 Mol-% an wiederkehrenden Struktureinheiten der Formel I enthalten,
wobei R¹ für die Verbindung der Formel II steht,
X für -NH- steht und
R² für
a) geradkettig oder verzweigtes Alkyl oder Alkenyl, mit 1 bis 18 C-Atomen oder
b) geradkettig oder verzweigtes Alkyl oder Alkenyl, mit 1 bis 18 C-Atomen, ein- oder mehrfach substituiert durch einen Rest aus der Gruppe:
   1) Carboxy oder
   2) Carboxy, worin die Hydroxygruppe ersetzt ist durch einen Rest aus der Gruppe:
      2.1 -O- (C₁-C₁₈)-Alkyl, geradkettig oder verzweigt,
      2.2 -O- (C₃-C₁₈)-Cycloalkyl,
      2.3 (C₁-C₆)-Alkylamino oder
      2.4 (C₁-C₆)-Alkylamino, ein- oder mehrfach substituiert im Alkylteil durch einen Rest aus der Gruppe:
         2.4.1 Hydroxy oder
         2.4.2 -O- (C₂-C₄)-Alkyl, steht.

Besonders bevorzugt werden in den Nanopartikeln Polykondensate der Formel I eingesetzt, die im wesentlichen mindestens 95 Mol-% an wiederkehrenden Struktureinheiten der Formel I enthalten,
wobei R¹ für die Verbindung der Formel II steht, X für -NH- steht und
R² für
a) Alkyl mit 1 bis 18 Kohlenstoffatomen,
b) (C₃-C₈)-Cycloalkyl oder
c) den Rest der Formel III steht, 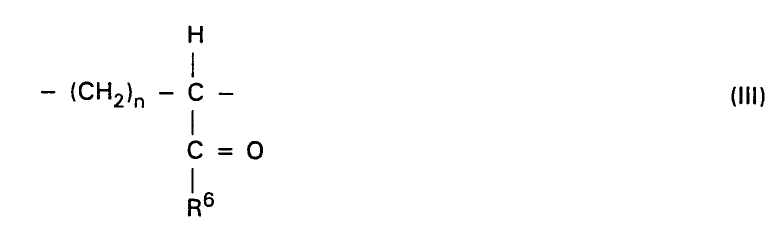 wobei n für 3 oder 4 und R⁶ für (C₁-C₁₈)-Alkyloxy steht.

Zur Herstellung der Polyketalweinsäureamide der Formel I setzt man 2,3-Isopropylidenweinsäuredimethylester mit einem oder mehreren der Diamine, der Formel IV

H₂N - R² - NH₂ (IV)

um,
wobei R² die obengenannte Bedeutung hat.

Es ist für den Fachmann selbstverständlich, daß die Summe aller von dem 2,3-Isopropylidenweinsäuredimethylester (A) abgeleiteten Struktureinheiten und die Summe aller von den verschiedenen Diaminen (B) abgeleiteten Struktureinheiten im wesentlichen gleich sind. Daher unterscheiden sich die Mengen von (A) und (B) maximal um etwa 1 %, vorzugsweise maximal um 0,2 %; insbesondere sind sie im Rahmen der praktischen Meß- und Dosierungsmöglichkeiten gleich.

Das Molekulargewicht der entstehenden Polyketalweinsäureamide läßt sich unter anderem über die Auswahl der Mengenverhältnisse von A zu B steuern. Diese Auswahlkriterien sind dem Fachmann auf dem Gebiet der Polykondensation bekannt.

Beispiele für geeignete Diamine der Formel IV sind 1,6-Diaminohexan, 1,8-Diaminooctan, Trans-1,4-Diaminocyclohexan oder 1,12-Diaminododecan.

Die Kondensation der oben beschriebenen Komponenten wird im allgemeinen in Lösung ausgeführt. Dazu werden die miteinander umzusetzenden monomeren Verbindungen in der Regel in einem organischen Lösungsmittel gelöst. Das organische Lösungsmittel enthält dabei vorzugsweise zumindest ein Lösungsmittel, z. B. N-Methyl-2-pyrrolidon oder Toluol.

Die Synthese der Polyketalweinsäureamide der Formel I erfolgt in zwei Stufen ausgehend von Weinsäure und Diamin. Die erste Stufe des Isopropylidenweinsäuredimethylesters kann ohne Isolierung der Zwischenstufe Isopropylidenweinsäure direkt aus Weinsäure und 2,2-Dimethoxypropan hergestellt werden.

Die Polykondensation von Isopropylidenweinsäuredimethylester mit Diaminen erfolgt unter Abspaltung von Methanol. Der Einsatz eines Katalysators oder Hilfsreagenzes ist hierzu nicht erforderlich. Da die exotherme Reaktion der Edukte bereits bei Raumtemperatur beginnt, ist zur Erreichung eines hohen Molekulargewichts auf die exakte Einhaltung der molaren Verhältnisse der Reaktanten zu achten.

Zur Durchführung der Lösungspolymerisation werden hochsiedende Lösungsmittel wie N-Methylpyrrolidon oder Toluol verwendet. Die Reaktion wird beispielsweise von 80 bis 140°C durchgeführt. Das entstehende Methanol wird azeotrop abdestilliert oder durch Einleitung eines N₂-Stroms entfernt.

Einen besonderen Vorzug bildet die Schmelzkondensation, die mit den Weinsäuremethylestern trotz der Ketalgruppen durchgeführt werden kann und in eleganter und einfacher Weise zu einheitlichen Produkten führt. Dazu werden die Reaktanten zunächst für 1 bis 6 Stunden (h) bei Temperaturen von 30 bis 80°C gerührt, um das Entweichen einer der Monomerkomponenten zu verhindern. Danach wird unter vermindertem Druck für 8 bis 16 h auf 80 bis 120°C erwärmt. Zur Aufarbeitung werden die Polymere üblicherweise in Dichlormethan gelöst und in geeigneten Fällungsmitteln wie Diisopropylether ausgefällt.

Im Verlauf der Polykondensation wächst das Molekulargewicht des Polymers und damit auch die Viskosität des Reaktionsansatzes an. Es werden Molekulargewichte von 1000 bis 100000 erreicht, bevorzugt 5000 bis 40000. Es werden in der Regel Viskositäten von mehr als 0,1 dl·g⁻¹ (Staudinger-Index, Dimethylformamid, 25 °C) erreicht.

Der Aufbau der Polyketalweinsäureamide ermöglicht eine gute Steuerung der Abbaubarkeit, da eine Hydrolyse sowohl über die Seitengruppen als auch über die Hauptkette erfolgen kann.

Die rigide Struktur der erhaltenen Polyketalweinsäureamide wird durch den Ketalring der Weinsäure hervorgerufen und verleiht den Polymeren die Eigenschaft trotz der wasserstoffbrückenbildenden Amidstruktur feste, in wasserunlösliche Formen, speziell Partikel, zu bilden, die sogar noch wasserlösliche Komponenten wie Proteine aufnehmen können, ohne daß die Form in weiche, klebrige Aggregate übergeht, wie es oft bei anderen Polyamiden beobachtet wird.
Es gelingt sogar, die Größe der Partikel bis in den Nanometerbereich zu verringern unter Erhalt der für Transportsysteme erforderlichen Eigenschaften, insbesondere der hohen Beladungskapazität, der geringen Aggregationsneigung in Lösung sowie der Stabilität der getrockneten Nanopartikel und deren Resuspendierbarkeit. Darüber hinaus können diese Nanopartikel weitere Komponenten mit sehr hydrophilen Gruppen wie Polyamine, Polyhydroxycarbonsäuren und polymere Fluoreszenzmarker aufnehmen ohne ihre definierte partikuläre Form zu verlieren.

Die Herstellung der Nanopartikel erfolgt durch Koazervation oder Sprühtrocknung.

Bei der Koazervation werden Polyketalweinsäureamide und Wirkstoffe getrennt gelöst, zu einer homogenen Lösung vereint und in einem Fällungsmittel zu Nanopartikeln ausgefällt. Dabei wird der Wirkstoff in die Polymermatrix eingeschlossen. Bei Verwendung von Peptid- und Protein-Wirkstoffen kommen Lösungs- und Fällungsmittel zur Anwendung, die zu keiner Beeinträchtigung des Wirkstoffs z.B. durch Denaturierung führen.

So wird eine 0,5- bis 10-%ige, vorzugsweise 3%ige Lösung der Polyketalweinsäureamide der Formel I in Aceton, Methanol oder Ethanol mit einer 1,5%igen Lösung eines Peptids oder Proteins , z.B. Insulin, in Methanol/Wasser oder Ethanol/Wasser mit einem pH von 2 bis 4, gemischt im Verhältnis 1:1 und bei einer Temperatur von 15 bis 40°C in ein 2- bis 10-faches, vorzugsweise 5-faches Volumen Wasser durch eine Kanüle mit einem Außendurchmesser von 0,2 bis 1,2 mm eingetragen. Das Ende der Kanüle befindet sich direkt oberhalb der Wasseroberfläche. Das Volumen der erhaltenen Tröpfchen beträgt 0,0005 bis 0,01 ml, bevorzugt 0,001 bis 0,005 ml. Dabei wird die Vorlage schnell gerührt, wobei sich das Rührorgan mit etwa 1000 Umdrehungen pro Minute (1000 rpm) dreht. Die erhaltene Nanopartikel -Suspension wird mit NaOH auf pH = 7 eingestellt und zentrifugiert. Die Nanopartikelfraktion wird in Wasser resuspendiert gegebenenfalls unter Zusatz eines Suspensionshilfsmittels (maximal 1 %), zentrifugiert und gefriergetrocknet. Die so erhaltenen getrockneten Nanopartikel lassen sich gut resuspendieren. Es zeigt sich, daß mit den Polyketalweinsäureamiden der Formel I der im allgemeinen besonders schwierige Schritt, der Isolierung der Nanopartikel aus der Suspension gut möglich ist, insbesondere wenn die beschriebenen Bedingungen eingehalten werden. Um Aggregatbildungen zu vermeiden, sollten die benutzten Löse- und Fällungsmittel nicht gekühlt werden und die Rührgeschwindigkeit deutlich unter 20000 rpm liegen.

Die Größe der Nanopartikel läßt sich über die Konzentration der Polymerlösung sowie über das Volumenverhältnis von Löse- zu Fällungsmittel einstellen. Als Ligand können Vitamin B12, Vitamin B12-Analoge wie Cyanocobalamin, Aquocobalamin, Adensosylcobalamin, Methylcobalamin, Hydroxycobalamin, Cyanocobalamincarbanalid, 5-O-Methylbenzylcobalamin, Desdimethyl-, Monoethylamid- und Methylamidanaloge dieser Verbindungen, Analoge und Homologe von Cobamamid, Coenzym B12, 5'-Deoxyadenosylcobalamin, Chlorocobalamin, Sulfitocobalamin, Nitrocobalamin, Thiocyanatocobalamin, Benzimidazolderivate, Adenosylcyanocobalamin, Cobalaminlacton, Cobalamin-lactam und die Anilid-, Ethylamid-, Propionamid-, Monocarbonsäure- und Dicarbonsäurederivate von Vitamin B12, Vitamin B12-Adipylhydrazid oder ihre Analogen oder Lektine wie Concanavalin A oder Weizenkeim-Lektin verwendet werden.

Als Kopplungsgruppen werden Polyvinylamin oder Polylysin verwendet, die mit freien Carboxyl- oder Amingruppen auf der Oberfläche des Nanopartikels kovalente Bindungen bilden. Ferner können der Ligand und die Kopplungsgruppe noch über eine Abstandshalteverbindung wie einen Alkylrest mit 1 bis 18 Kohlenstoffatomen, bevorzugt 4 bis 8 Kohlenstoffatomen, miteinander verbunden sein. Bevorzugt wird Vitamin B12-Monocarboxylsäure über ein Adipylhydrazid an die Nanopartikel gekoppelt. Zur Herstellung von Nanopartikeln, die zur Funktionalisierung koppelbare Einheiten auf der Oberfläche aufweisen, werden Polyamine, wie Polyvinylamin oder Polylysin, und/oder Polyhydroxysäuren wie Polyasparaginsäure zu den Polyketalweinsäureamiden der Formel I in Konzentrationen von 1 bis 25% zugegeben. Die weitere Bearbeitung erfolgt wie oben beschrieben.

Zur Herstellung von Nanopartikeln mittels Sprühtrocknung werden die zu versprühenden Polyketalweinsäureamide in Konzentrationen von 0,2 bis 5 %, vorzugsweise 0,5 bis 1,5 % in reinen Lösungsmitteln wie Aceton, Methanol, Ethanol oder Dioxan sowie in Mischungen dieser Komponenten unter gegebenenfalls geringem Zusatz von Wasser angesetzt. Diesen Polymerlösungen werden die Wirkstoffe in Konzentrationen von 1 bis 60 %, vorzugsweise von 4 bis 25 % (w/w bezogen auf Polyketalweinsäureamid), gegebenenfalls FITC-markiertes Polymer (0,2 bis 5 %), oder Suspensionshilfsmittel im Bereich von 1 bis 20%, vorzugsweise von 2 bis 10%, sowie basiches Polymere im Bereich von 0,5 bis 30 %, insbesondere 1 bis 10 %, zugesetzt.

Für die Herstellung von wirkstoffbeladenen Nanopartikeln mit einer Größe von weniger als 1000 nm muß die zu versprühende Lösung aus Polymer, Wirkstoff und gegebenenfalls weiteren Zusätzen eine spezifische Viskosität von 0,2 bis 3,5 , insbesondere 0,3 bis 2,0 aufweisen.

Versprüht wird bei einer Eingangstemperatur des Sprühtrockners von 40 bis 110 °C vorzugsweise unterhalb von 80 °C, einer Ausgangstemperatur von 25 bis 70 °C, vorzugsweise von 30 bis 50 °C einer Flow-Rate N₂ von 500 l/h bis 800 l/h und einem Volumenstrom von 1 bis 5 ml/min.

Die entstandenen Nanopartikel werden an einem engmaschigen Siebgewebe (Nylongewebe mit einer Porengröße von 0,010 mm) aufgefangen. Die Partikelgröße beträgt von 10 nm bis 1000 nm, der Mittelwert liegt in der Regel deutlich unter 600 nm. Je nach Zusammensetzung werden die Partikel nach dem Versprühen für 2 bis 8 h unter vermindertem Druck aufbewahrt. Die Ausbeute beträgt 25 bis 80 %, in der Regel mehr als 50 %.

Die erhaltenen Nanopartikel nach der Sprühtrocknungs- oder Koazervationsmethode können zur Reduktion von größeren Partikeln noch filtriert werden. Geeignete Filter haben eine Porenweite von 0,2 µm bis 0,8 µm, bevorzugt 0,45 µm. Geeignete Membranen bestehen beispielsweise aus Celluloseacetat.

Die Partikelgröße wird mittels Photonenkorrelationsspektroskopie (PCS) Aerosolspektroskopie und Elektronenmikroskopie (REM ) bestimmt (J.P. Fischer, Progress in Colloid and Polymer Science, 77, Seiten 180-194, 1988), falls vorhanden wird die Oberflächenbasizität der Nanopartikel mittels Polyelektrolyt-Titration (Streaming current detector, SCD), der Fluoreszenzgehalt spektroskopisch und der Wirkstoffgehalt und - release chromatographisch bestimmt. Die erhaltenen Nanopartikel weisen eine enge Größenverteilung auf. Die Uneinheitlichkeit der erhaltenen Nanopartikel (dw/dn) beträgt von 1,1 bis 2,0, insbesondere 1,15 bis 1,5. Aufgrund der engen Größenverteilung eignen sich die Nanopartikel auch zu Eichzwecken bei der Rasterelektronenmikroskopie oder zum Nachweis der Verteilung von Wirkstoffen im Körper von Tieren oder Menschen nach oraler Applikation, wenn die Nanopartikel einen fluoreszierenden oder radioaktiven Marker enthalten. Das biologisch abbaubare Partikel zerfällt und wird vom Körper ausgeschieden.

### Beispiel 1

### Poly-(2,3-O-isopropyliden)-DL-weinsäure-1',6'-hexylamid

Synthese von Dimethyl-2,3-O-isopropyliden-DL-tartrat 700 g DL-Weinsäure und 7 g p-Toluolsulfonsäure werden in 1 L (Liter) Methanol unter Erwärmung gelöst und mit 1,2 L 2,2-Dimethoxypropan so langsam versetzt, daß die Weinsäure nicht mehr ausfällt. Der Ansatz wird 5 h unter Rückfluß gekocht. Danach wird das entstandene Aceton kontinuierlich und langsam abdestilliert (Destillat: 53% Aceton, 38% Methanol, 11% Dimethoxypropan). Es werden zum Ansatz weitere 320 ml 2,2-Dimethoxypropan hinzugefügt und die Weinsäure vollständig zum Dimethylester umgesetzt. Nach Beendigung der Reaktion (insgesamt 15 h) wird das Methanol mit den Resten von Dimethoxypropan und Aceton abdestilliert. Zum Rückstand werden 700 ml 2,2-Dimethoxypropan und 1 L Cyclohexan gegeben. Das durch Bildung von Methanol entstehende Azeotrop mit Cyclohexan wird bei 54°C sehr langsam und kontinuierlich abdestilliert.

Portionsweise werden 540 ml 2,2-Dimethoxypropan und 820 ml Cyclohexan nachdosiert. Nach Beendigung der Reaktion (DC-Kontrolle) wird der Ansatz mit 10 g getrocknetem Kaliumcarbonat neutralisiert und fraktioniert destilliert. Das Produkt geht bei 95°C und 0,1 mbar über.
Ausbeute: 972 g

### Poly-(2,3-O-isopropyliden)-D,L-weinsäure-1',6'-hexylamid

1,6-Diaminohexan wird aufgeschmolzen und im exakten molaren Verhältnis (7,814 g, 60 mmol) zu Dimethyl-2,3-O-isopropyliden-D,L-tartrat (13,094 g, 60 mmol) eingewogen. Nach Zugabe von 15 ml N-Methylpyrrolidon wird der Ansatz unter N₂ 4 Tage bei 120°C gerührt. Während dieser Zeit werden 5 mal je 20 ml Toluol zugegeben und zusammen mit dem gebildeten Methanol abdestilliert. Nach Zugabe von Dichlormethan wird in eisgekühltem Diisopropylether 2 mal gefällt. Das gebildete Polymer wird unter vermindertem Druck bei 40°C bis zur Gewichtskonstanz getrocknet.
Ausbeute: 13,7 g
Erweichungspunkt T_{g}: 96°C

### Beispiel 2

### Poly-2,3-O-isopropyliden-L-weinsäure-1',4'-cyclohexylamid-co-1',12'-dodecylamid (1:1)

Trans-1,4-Diaminocyclohexan (2,853 g, 24.98 mmol), 1,12-Diaminododecan (5,00 g, 24,98 mmol) und Dimethyl-2,3-O-isopropyliden-L-tartrat (10.91 g, 49,97 mol) werden direkt im Laborreaktor eingewogen. Unter einem leichten N₂-Strom wird langsam auf 80°C erwärmt und 2,5 h bei dieser Temperatur gerührt. Dabei destilliert das gebildete Methanol ab. Bei Rührgeschwindigkeiten von 10 bis 60 rpm wird die Temperatur auf 130°C erhöht und der Druck für 4 h auf 200 mbar und für weitere 4 h auf 0,5 mbar reduziert. Das gebildete Polymer wird in Dichlormethan gelöst, 3 mal in Diisopropylether gefällt und danach unter vermindertem Druck getrocknet.
Erweichungspunkt T_{g}: 125°C

### Beispiel 3

### Poly-2,3-O-isopropyliden-L-weinsäure-1',8'-octylamid

Dimethyl-2,3-O-isopropyliden-L-tartrat (36,66 g, 168 mmol) und frisch sublimiertes 1,8-Diaminooctan (24,24 g, 168 mmol) werden direkt im Laborreaktor eingewogen. Unter einem leichten N₂-Strom wird langsam auf 80°C erwärmt und 2,5 h bei dieser Temperatur gerührt. Dabei destilliert das gebildete Methanol ab. Bei Rührgeschwindigkeiten von 10 bis 60 rpm wird der Druck bei 100°C für 4 h auf 200 mbar und für weitere 4 h auf 0,5 mbar reduziert. Das Produkt wird in Dichlormethan gelöst, 3 mal in Diisopropylether gefällt und danach unter vermindertem Druck getrocknet.
Ausbeute: 47,3 g
Erweichungspunkt T_{g}: 104°C

### Beispiel 4

Mit dem Polymer aus Beispiel 3 wird eine 3%ige Lösung in 1 ml Methanol hergestellt. Es werden 15 mg Insulin unter Verwendung von HCl (0,1 molar) bei pH = 2,7 und nachfolgender pH-Einstellung (mit 0,1 N NaOH) auf 7,5 in einem Gesamtvolumen von 1 ml in Methanol gelöst. Die beiden Lösungen werden zusammengegeben und durch eine Kanüle (26G*23, Luer) mit einem Außendurchmesser von d = 0,45 mm in 8 ml destilliertes Wasser mit einer Geschwindigkeit von 0,5 ml/min eingetropft.

Die Suspension wird bei 15 000 rpm 15 min zentrifugiert, der Überstand wird dekantiert und die Partikelfraktion in 10 ml Wasser aufgenommen. Die Suspension wird erneut unter den obengenannten Bedingungen zentrifugiert und nach Resuspension gefriergetrocknet. Ausbeute: 48%
Partikeldurchmesser: kleiner als 500 nm (gemäß REM)

### Beispiel 5

In einem Sprühtrockner (Mini Spray Dryer, Büchi) wird eine 0,5%ige Lösung versprüht, die aus drei Einzellösungen besteht, die in der Reihenfolge 1 bis 3 zusammengegeben werden:

| | | |
|---|---|---|
| Lösung 1: | 280 mg Polymer (Beispiel 3), | gelöst in 55 ml Methanol |
| Lösung 2: | 60 mg Polylysin | gelöst in 10 ml Methanol |
| Lösung 3: | 40 mg Insulin | gelöst in 10 ml Methanol und 0,040 ml 1N HCl |

Diese Lösung wird unter folgenden Parametereinstellungen versprüht:

| | |
|---|---|
| Inlet Temperatur | 72 °C |
| Outlet Temperatur | 45 °C |
| Pumpenrate | 3 ml/min |
| Aspirator | 10 |
| Heizung | 2,9 |
| Flow | 700 |

Partikeloberflächenbasizität: 70% der eingesetzten Aminogruppen sind titrierbar. Durchmesser der resuspendierten Nanopartikel: 330 nm (gemäß PCS)

### Beispiel 6

Versprüht wird eine Mischung aus den folgenden Lösungen 1 und 2:

| | |
|---|---|
| Lösung 1: | 25 mg Polymer (gemäß Beispiel 3) |
| | gelöst in 10,4 ml Methanol |
| Lösung 2: | 1,56 mg Insulin |
| | gelöst in 1,04 ml Methanol und 0,01 ml 1N HCl |

Parametereinstellung am Sprühtrockner:

| | |
|---|---|
| Inlet Temperatur | 78 °C |
| Outlet Temperatur | 49 °C |
| Pumpenrate | 2 ml/min |
| Aspirator | 10 |
| Flow | 800 |

Durchmesser der resultierenden Nanopartikel (dn) 56 nm (gemäß PCS); dw = 80; dw/dn = 1,43

### Beispiel 7

### Herstellung von Vitamin B12-N-hydroxy-succinimidyl-ester

Die Herstellung eines Monocarboxyl-Derivats von Vitamin B12 erfolgt durch saure Hydrolyse und Reinigung über einen Ionenaustauscher wie DOWEX® AGI-X8 (EP 0 220 030). 50 mg der erhaltenen Monocarboxylsäure von Vitamin B12 werden in 500 µl DMSO gelöst und anschließend werden hierzu 15 µl Diisopropylethylamin gegeben. Die Lösung wird bei Raumtemperatur unter N₂ Schutzgas gerührt. Zu dieser Lösung gibt man eine Lösung von 18 mg TSTU (O-(N-Succinimidyl)-N,N,N',N'-tetramethyluronium-tetrafluoroborat) in 100 µl DMF. Die Reaktionslösung läßt man bei Raumtemperatur für 20 Minuten reagieren. Zur Aufarbeitung wird die Reaktionslösung mit 25 ml destilliertem Wasser verdünnt und durch einen 0,45 µm Filter (Schleicher & Schuell Einmal-Filtrationseinheit, Celluloseacetat-Membran) filtriert und mittels einer präperativen Reverse-Phase-Hochdruckflüssigchromatographie-säule (RP-HPLC- Säule; Vydac 218TP1010; mobile Phase A: 0,1% Trifluoressig-säure (TFA) in dest. Wasser, mobile Phase B: 0,1% TFA in Acetonitril: Wasser (70:30), Fluß: 4ml/min) getrennt. Die Produktfraktion wird nach 20-22 Minuten eluiert und wird anschließend sofort eingefroren und lyophilisiert.

### Beispiel 8

### Filtration

440 mg Nanopartikel, hergestellt gemäß Beispiel 4, werden in 10 ml destilliertem Wasser suspendiert und durch ein Einmalfilter mit Celluloseacetat-Membran und der Porenweite von 0,45 µm filtriert. Ausbeute: 385 mg, durchschnittlicher Partikeldurchmesser 320 nm.

### Beispiel 9

### Koppelung von Vitamin B12 an Nanopartikel

20 mg Nanopartikel, hergestellt gemäß Beispiel 5, werden in 300 µl destilliertem Wasser suspendiert. 3 mg von VB₁₂-TSTU-Ester, gemäß Beispiel 7, werden in 50 µl destilliertem Wasser gelöst. Zu der Suspension wird 0,01 N NaOH zugesetzt bis ein pH-Wert von 7 +/-0,2 erreicht ist. Nach diesem Schritt wird der VB₁₂-TSTU-Ester zugeführt. Die Suspension wird 6 +/- 0,5 Stunden (h) bei Raumtemperatur gerührt. Die Suspension wird mit 15 ml destilliertem Wasser verdünnt und die Reinigung erfolgt durch wiederholte Filtrationen (0,22 µm Celluloseacetat Membran, 0,8 bar (N₂) und mittlerer Rührgeschwindikeit) mit 0.5% in Wasser gelöstem Polyvinylpyrrolidon 90 (BASF) (PVP90) als Waschlösung bei einer Temperatur von 2 bis 8°C. Die Reinigung kann beendet werden, wenn das Filtrat keine schwache Rotfärbung mehr aufweist. Die Suspension wird gefriergetrocknet und bei 2 bis 8°C bis zur weiteren Verwendung gelagert. Der Gehalt an Vitamin B12 an der Oberfläche der Nanopartikel wird durch "Elektronspectroscopy for chemical application" mit 0,03 Atomprozent Kobalt bestimmt.

### Beispiel 10

### Kopplung von H₂NNH-C(O)-(CH₂)₄-C(O)-NHNH-C(O)-Vitamin B₁₂ an Nanopartikel

### Herstellung von Vitamin B12-Monocarbonsäure-adipylhydrazid

Adipinsäuredihydrazid und Vitamin B12-Monocarbonsäure werden analog zu Beispiel 7 umgesetzt. Die Reinigung erfolgt über Hochdruckflüssigchromatographie analog zu Beispiel 7.

20 mg Nanopartikel, hergestellt gemäß Beispiel 5, werden in 500 µl DMSO suspendiert und anschließend werden 15 µl Diisopropylethylamin zugegeben. Die Lösung wird bei Raumtemperatur unter N₂ Schutzgas gerührt. Zu dieser Lösung gibt man eine Lösung von 18 mg TSTU (O-(N-Succinimidyl)-N,N,N',N'-tetramethyluronium-tetrafluoroborat), hergestellt gemäß Beispiel 7, gelöst in 100 µl Dimethylformamid (DMF). Die Reaktionslösung läßt man bei Raumtemperatur für 20 Minuten reagieren.

10 mg Vitamin B12-monocarbonsäure-adipylhydrazid ( wie oben hergestellt) werden in 100 µl destilliertem Wasser gelöst und zu der obigen Reaktionslösung gegeben. Man läßt 6 Stunden bei Raumtemperatur rühren. Anschließend wird die Suspension mit 15 ml dest. Wasser verdünnt und die Reinigung erfolgt durch wiederholte Filtration (0,22 µm Millipore Filter, 0,8 bar N₂ und mittlerer Rührgeschwindikeit) mit 0,5% PVP90 als Waschlösung bei 2 bis 8°C. Das Retentat wird lyophilisiert und bei 2 bis 8°C gelagert.

### Beispiel 11

140 g Anhydroasparaginsäure werden in 800 ml Wasser suspendiert. Über einen Zeitraum von 5 Stunden werden 350 ml 2 molar Natronlauge so zugetropft, daß der pH-Wert nicht über 9,5 +/- 0,1 ansteigt. Die Reaktionslösung wird filtriert und der pH-Wert des Filtrates wird auf 7 eingestellt. Die im Filtrat enthaltene Polyasparaginsäure wird mehrfach durch Ultrafiltration gereinigt und anschließend lyophilisiert.

In einem Sprühtrockner (Mini Spray Dryer, Büchi) wird eine 0,5%ige Lösung versprüht, die aus drei Einzellösungen besteht, die in der Reihenfolge 1 und 2 zusammengegeben werden:

| | |
|---|---|
| Lösung 1: | 1 g Polymer (gemäß Beispiel 3) |
| | 12 mg Polyvinylamin |
| | 12 mg Polyasparaginsäure (wie oben hergestellt) |
| | Lösungsmittel 140 ml Methanol und 40 ml Dioxan |
| Lösung 2: | 60 mg Insulin |
| | Lösungsmittel 20 ml Methanol und 0,060 ml 1 N HCl |

Diese Lösung wird unter folgenden Parametereinstellungen versprüht:

| | |
|---|---|
| Inlet Temperatur: | 80°C |
| Outlet Temperatur: | 43°C |
| Pumpenrate: | 2,5 ml/min |
| Aspirator: | 10 |
| Flow: | 800 |

Durchmesser der resuspendierten und anschließend filtrierten Nanopartikel beträgt 160 nm (gemäß PCS).

## Patentansprüche

1. Nanopartikel, enthaltend einen Wirkstoff und ein Polyketalweinsäureamid, das mindestens 95 Mol-% an wiederkehrenden Struktureinheiten der Formel I enthält, 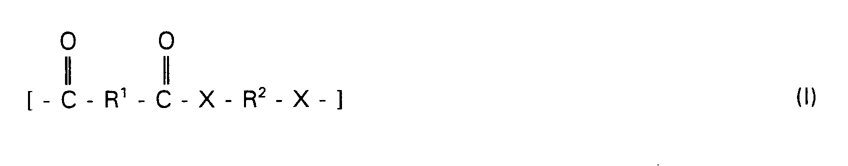 wobei R¹ für den Rest der Formel II 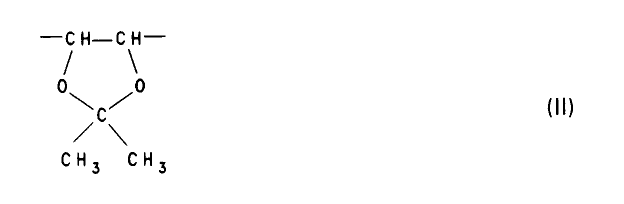 steht,
X für -NH- steht und
R² für geradkettiges oder verzweigtes Alkyl oder Cycloalkyl steht, das mit einem oder mehreren inerten Resten substituiert sein kann.

2. Nanopartikel, enthaltend ein Polyketalweinsäureamid der Formel I nach Anspruch 1, wobei R¹ für den Rest der Formel II steht, X für -NH- steht und R² für
a) geradkettig oder verzweigtes Alkyl oder Alkenyl, mit 1 bis 18 C-Atomen oder
b) geradkettig oder verzweigtes Alkyl oder Alkenyl, mit 1 bis 18 C-Atomen, ein- oder mehrfach substituiert durch einen Rest aus der Gruppe:
1) Carboxy oder
2) Carboxy, worin die Hydroxygruppe ersetzt ist durch einen Rest aus der Gruppe:
2.1 -O- (C₁-C₁₈)-Alkyl, geradkettig oder verzweigt,
2.2 -O- (C₃-C₁₈)-Cycloalkyl,
2.3 (C₁-C₆)-Alkylamino oder
2.4 (C₁-C₆)-Alkylamino, ein- oder mehrfach substituiert im Alkylteil durch einen Rest aus der Gruppe:
2.4.1 Hydroxy oder
2.4.2 -O- (C₂-C₄)-Alkyl, steht.

3. Nanopartikel, enthaltend ein Polyketalweinsäureamid nach Anspruch 1 oder 2, wobei R¹ für den Rest der Formel II steht,
X für -NH- steht und
R² für
a) Alkyl mit 1 bis 18 C-Atomen,
b) (C₃-C₈)-Cycloalkyl oder
c) den Rest der Formel III steht, 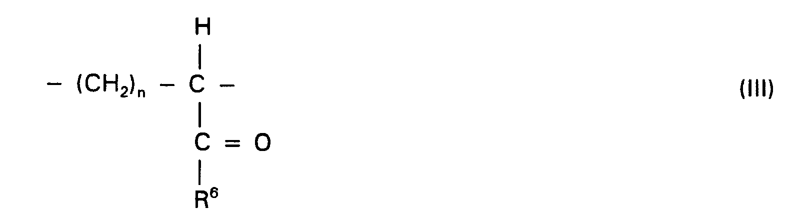 wobei n für 3 oder 4 und R⁶ für (C₁-C₁₈)-Alkyloxy steht.

4. Nanopartikel, nach einem oder mehreren der Ansprüche 1 bis 3, dadurch gekennzeichnet, daß der Durchmesser 10 bis 1000 nm, insbesondere 200 bis 600 nm, beträgt.

5. Nanopartikel, nach einem oder mehreren der Ansprüche 1 bis 4, dadurch gekennzeichnet, daß der Wirkstoffgehalt bezogen auf das Polyketalweinsäureamid 1 bis 60 % beträgt.

6. Nanopartikel, nach einem oder mehreren der Ansprüche 1 bis 5, dadurch gekennzeichnet, daß es als Wirkstoff Insulin, Calcitonin oder Buserelin enthält.

7. Nanopartikel, nach einem oder mehreren der Ansprüche 1 bis 5, dadurch gekennzeichnet, daß an das Nanopartikel Liganden aus der Gruppe Vitamin B12, Vitamin B12-Analoge und Lektine gebunden sind.

8. Nanopartikel, nach Anspruch 7, dadurch gekennzeichnet, daß die Liganden über Kopplungsgruppen aus der Gruppe Polyvinylamin, Polyasparaginsäure, Polylysin und O-(N-Succinimidyl)-N,N,N',N'-tetramethyluronium-tetrafluoroborat an das Nanopartikel gebunden sind.

9. Verfahren zur Herstellung der Nanopartikel nach Anspruch 1, dadurch gekennzeichnet, daß man
a) den Wirkstoff und das Polyketalweinsäureamid der Formel I und gegebenenfalls weitere Hilfsstoffe getrennt löst, die erhaltenen Lösungen mischt und durch eine Kanüle mit einem Außendurchmesser von 0,2 bis 1,2 mm in ein Fällungsmittel einträgt oder
b) den Wirkstoff und das Polyketalweinsäureamid der Formel I und gegebenenfalls weitere Hilfsstoffe getrennt löst, die erhaltenen Lösung von 0,2 bis 3,5 beträgt, und die erhaltene Lösung sprühtrocknet,
c) die nach a) oder b) erhaltenen Nanopartikel an einen oder mehrere Liganden bindet,
d) die nach a) oder b) erhaltenen Nanopartikel über eine Kopplungsgruppe mit einen oder mehreren Liganden verbindet,
e) die nach a), b), c) oder d) erhaltenen Nanopartikel durch einen Filter mit einer Porengröße von 0,2 µm bis 0,8 µm, bevorzugt 0,45 µm, filtriert.

10. Verwendung der Nanopartikel nach einem oder mehreren der Ansprüche 1 bis 8 zur Herstellung von Arzneimittelzubereitungen mit kontrollierter Wirkstoffabgabe, die oral verabreicht werden.
